# EUROPEAN PATENT SPECIFICATION

(11) **EP 2 137 206 B1**
(45) Date of publication and mention of the grant of the patent: **06.01.2016**
(21) Application number: 08735477.5
(22) Date of filing: 26.03.2008
(51) Int. Cl.: C07J 41/00

(54) **PROCESS FOR THE PREPARATION OF TAUROURSODESOXYCHOLIC ACID**
VERFAHREN ZUR HERSTELLUNG VON TAUROURSODESOXYCHOLSÄURE
PROCÉDÉ DE PRÉPARATION DE L'ACIDE TAUROURSODÉSOXYCHOLIQUE

(30) Priority: 23.04.2007 EP 07425243
(43) Date of publication of application: 30.12.2009
(73) Proprietor: PRODOTTI CHIMICI E ALIMENTARI SPA, 15060 Basaluzzo (Alessandria) (IT)
(72) Inventor: PARENTI, Massimo, I-15067 Novi Ligure (al) (IT)
(74) Representative: Pistolesi, Roberto
(86) International application number: PCT/EP2008/053531
(87) International publication number: WO 2008/128844

(56) References cited:
- EP-A- 0 272 462
- EP-A- 0 629 634
- BE-A1- 901 069
- DE-A1- 3 736 918
- JP-A- 3 027 396
- JP-A- 7 267 985
- KR-B1- 100 396 113
- ZHUO CHAO ET AL: "Synthesis of tauroursodeoxycholic acid" HECHENG HUAXUE, ZHONG-KE-YUAN CHENGDU YOUJI HUAXUESUO TASHU QINGBAOSHI,, CN, vol. 10, no. 5, 2002, pages 444-446, XP008085059 ISSN: 1005-1511

## Description

The present invention relates to a novel method for preparing tauroursodeoxycholic acid, a substance useful in the treatment of biliary disorders and dysfunction, which method comprises a step of selective precipitation of the impurities present in the suspension obtained from the reaction of an aqueous solution of sodium taurinate with an acetonic solution of a mixed anhydride of ursodeoxycholic acid with an alkyl chloroformate.

### PRIOR ART

Italian patent IT1197330 describes the preparation of a mixed anhydride of tauroursodeoxycholic acid by reaction between ursodeoxycholic acid and an alkyl chloroformate in the presence of a tertiary base, which is reacted with an alkaline aqueous solution of taurine to obtain the sodium salt of tauroursodeoxycholic acid. Subsequent addition of hydrochloric acid and removal of the solvents by vacuum concentration make it possible to obtain a residue containing tauroursodeoxycholic acid. Resuspending the residue with ethanol and subsequent filtration remove the unreacted taurine and sodium chloride, while the tauroursodeoxycholic acid remains in solution. The latter is then precipitated out from the solution by addition of a solvent in which said acid is insoluble. The product is finally dissolved in water and reprecipitated in the solvent in order to reduce the solvent content in the precipitate.

European patent application EP400695 describes the preparation of tauroursodeoxycholic acid by means of a purification process of the solution obtained by reaction between taurine and a mixed anhydride of ursodeoxycholic acid in the presence of a tertiary base. In order to avoid the addition of a mineral acid (such as hydrochloric acid) to the sodium salt of tauroursodeoxycholic acid, a procedure which entails various purification steps, the solution arising from the reaction between taurine and the mixed anhydride is passed in series through a first strong cationic exchange resin and through a second weak anionic exchange resin.

In this manner, the unreacted tertiary base and the hydrochlorides are removed, while the unreacted taurine and ursodeoxycholic acid arc eliminated by known methods. Despite this method not involving the addition of mineral acid to liberate the free tauroursodeoxycholic acid, it does not do without the final purification sequence already described in IT1197330, i.e. concentration to dryness of the percolated solution, resuspension with ethanol, filtration and precipitation with acetone, redissolution in water and reprecipitation in acetone.

European patent application EP629634 discloses a process for producing tauroursodeoxycholic acid which requires huge amounts of organic solvent and the use of cationic resins.

European patent application EP272462 discloses a process for producing the calcium salt of the taurinamidc of ursodeoxycholic acid.

Italian patent IT1167038 discloses a process for producing tauroursodeoxycholic acid which comprises the preparation of a basic solution of a sodium salt of tauroursodeoxycholic acid, the dissolution of said sodium salt in methanol and the treatment of the thus-obtained methanol solution with gaseous HCl at 5° C.

Belgian patent application BE901069 discloses a process for producing tauroursodeoxycholic acid, which comprises the preparation of a basic solution of sodium salt of tauroursodeoxycholic acid, subsequent hydrolysis in methanol with HCl gas, filtration of NaCl and precipitation of tauroursodeoxycholic acid via the addition of ethyl acetate.

A requirement thus remains for a process for producing tauroursodeoxycholic acid which makes it possible to obtain a product in high yield with the lowest possible content of impurities, operating at room temperature and with low cost and easy to handle solvents, reactants and equipments.

### DESCRIPTION OF THE INVENTION

It has surprisingly been found that, by adding a specific number of equivalents of an acid to the alkali or alkaline-earth tauroursodeoxycholate suspension, subsequently leaving the resultant suspension to stand for a defined period, filtering the suspension and adding an organic solvent, it is possible selectively to precipitate the tauroursodeoxycholic acid in particularly pure form.

The present invention accordingly provides a process for preparing tauroursodeoxycholic acid comprising the following steps:
a) obtaining an aqueous suspension of an alkali or alkaline-earth salt of tauroursodeoxycholic acid;
b) adding 0.8-1.4 equivalents of an acid;
c) leaving the resultant suspension to stand for a period of between 10 and 180 minutes;
d) filtering the suspension;
e) adding an organic solvent with subsequent precipitation of the tauroursodeoxycholic acid.

An aqueous suspension of the alkali or alkaline-earth salt of tauroursodeoxycholic acid, preferably the sodium salt, is obtained in the presence of a tertiary base, by reaction between a mixed anhydride of ursodeoxycholic acid with an alkyl chloroformate, preferably ethyl chloroformate, and an alkali or alkaline-earth salt of taurine, preferably a sodium salt. From 0.8 to 1.4 equivalents of an acid are added to said suspension in order to precipitate the taurine and the alkali or alkaline-earth hydrochloride. Preferably from 0.9 to 1.2 equivalents and still more preferably from 1 to 1.1 equivalents of the acid are added. The acid is selected from among hydrochloric acid, nitric acid, acetic acid, sulfuric acid or mixtures thereof, with hydrochloric acid being particularly preferred. The concentration of the acid in water is greater than 30% by weight, with a concentration in water of between 32% and 40% by weight being preferred and a concentration in water of between 32% and 36% by weight being still more preferred. The resultant solution is left to stand for a period of between 10 and 180 minutes, at a temperature of preferably between 15 and 30°C, in order to achieve selective precipitation of the taurine and the alkali or alkaline-earth hydrochloride. In particular, periods of between 15 and 120 minutes are preferred, with periods of between 20 and 60 minutes being still more preferred.

Once the mixture of taurine and alkali or alkaline-earth hydrochloride has been filtered out, the tauroursodeoxycholic acid is precipitated by adding an organic solvent. Said organic solvent preferably has a water content of less than or equal to 1% by weight. The organic solvent is preferably a polar organic solvent, such as acetone, tetrahydrofuran, C₂-C₈ ethers, C₂-C₈ acetates and mixtures thereof. The C₂-C₈ ether is preferably ethyl ether and the C₂-C₈ acetate is preferably ethyl acetate. The particularly preferred solvent is acetone. Alternatively, the organic solvent may be a nonpolar organic solvent, such as chloroform, methylene chloride or mixtures thereof.

The tauroursodeoxycholic acid crystallises with 2 moles of water and many parts per million (ppm) of the organic solvent used for precipitation remain trapped in this water of crystallisation.

An additional purification step is thus carried out by dissolving the resultant tauroursodeoxycholic acid in deionised water. Dissolution is preferably carried out in a maximum period of 60 minutes and at a temperature of below 65°C. In order to avoid product losses, it is necessary to use the least possible quantity of water for dissolution; the aqueous solution is thus heated to a temperature such that all the acid passes into solution. It has accordingly been observed that the best ratio by weight of tauroursodeoxycholic acid:water is between 0.1 and 1, preferably between 0.4 and 0.6, and still more preferably approx. 0.48, relative to the dry weight of tauroursodeoxycholic acid.

When all the tauroursodeoxycholic acid has dissolved, the solution is placed under a vacuum such that the temperature drops sufficiently rapidly with the solvent dissolved in the solution being distilled off.

Wet yield is between 90 and 110% of the ursodeoxycholic acid introduced into the reaction for two operations. After vacuum drying, the yield of dried product is between 68% and 82% of the quantity of ursodeoxycholic acid introduced into the reaction.

According to a preferred embodiment of the invention, the aqueous suspension of the alkali or alkaline-earth salt of tauroursodeoxycholic acid is obtained from the reaction of an aqueous solution of alkali or alkaline-earth metal taurinate with an acetonic solution of a mixed anhydride of ursodeoxycholic acid with a C₁-C₄ alkyl chloroformate; preferably, the alkali metal taurinate is sodium taurinate and/or the C₁-C₄ alkyl chloroformate is ethyl chloroformate.

The process of the present invention permit to obtain a higly pure product in high yields, without requiring the use of exchange resins (such as cationic resins); furthermore, it is carried out at room temperature and with low cost and easy to handle solvents (such as water) and reactants (such as aqueous acids). In particular, the process of the present invention allows to obtain crude TUDCA from the aqueous solution of its alkali or alkaline-earth metal salt, by simple acidification with mineral acids, since impurities like chloride and taurine are water soluble.

The following Examples describe the present invention in greater detail, without there being any intention to restrict the scope thereof.

### EXAMPLE 1

### Preparation of a sodium tauroursodeoxycholate suspension

A solution of sodium taurinate is prepared by introducing 95.6 kg of taurine and 30.9 kg of caustic soda flakes with a minimum content of 97%. 186 kg of demineralised water are added and the suspension is stirred for approx. 30 minutes while maintaining a temperature of approx. 15°C. In this manner, the sodium taurinate solution is obtained.

The mixed anhydride of tauroursodeoxycholic acid is then synthesised starting from 250 kg of ursodeoxycholic acid, 64.9 kg of triethylamine and 69.4 kg of ethyl chloroformate. The ursodeoxycholic acid and 1500 litres of acetone are introduced into a reactor at a temperature of less than 10°C. Under the residual vacuum, the triethylamine is added to the suspension and the temperature is reduced to < 0°C. When the temperature of the suspension is < 0°C, the ethyl chloroformate is introduced over approx. 15 minutes. Once addition is complete, the resultant mixed anhydride is stirred for approx. 20 minutes. The triethylamine hydrochloride is then centrifuged and washed with acetone.

The solution of sodium taurinate is introduced into the reactor containing the mixed anhydride.

### Preparation of crude tauroursodeoxycholic acid (TUDCA)

Approx. 30 minutes after the end of hydrolysis, 87 kg of concentrated (33% wt./wt.) hydrochloric acid solution are added. Stirring of the suspension is continued for 60 minutes. Precipitation of the sodium chloride and the taurine takes place. The suspension is centrifuged.

Approx. 2300 litres of acetone are added to the filtered solution. After approx. 60 minutes, the crude TUDCA starts to precipitate out.

Stirring is continued for 12 hours.

The crude TUDCA is separated by centrifugation.

Wet yield approx. 350 kg.

Determination of the chromatographic quality of the crude TUDCA reveals the following results:
impurities <0.3%; taurine <0.5%; ursodeoxycholic acid <0.5%, chloride < 1%.

### EXAMPLE 2

### Preparation of pure tauroursodeoxycholic acid

1100 litres of demineralised water are introduced into a reactor and are heated, with stirring, up to approx. 80°C. 695 kg of wet crude tauroursodeoxycholic acid, obtained according to Example 1, are introduced into a second reactor and the 1100 litres of water which have previously been heated are introduced therein. The suspension is heated to a temperature of 60°C. At this point, the reactor is slowly evacuated. Once no further acetone is distilled off, distillation is terminated, the vacuum is replaced with nitrogen and the solution is cooled to approx 0°C.

Stirring of the suspension is continued for 64 hours at a temperature of between 0 and 5°C.

The purified TUDCA is then recovered by centrifugation, the product being washed with approx 50 litres of demineralised water for each centrifuge batch. Wet yield 400 kg.

The wet product is then dried under a vacuum at a maximum temperature of 50°C. The chromatographic quality of the purified TUDCA exhibits the following properties:
taurine <0.2%; ursodeoxycholic acid <0.5%; impurities <0.3%, chloride < 500 ppm, acetone <500 ppm.
Content by HPLC is greater than 98.5%.

## Claims

1. A process for preparing tauroursodeoxycholic acid having the following formula 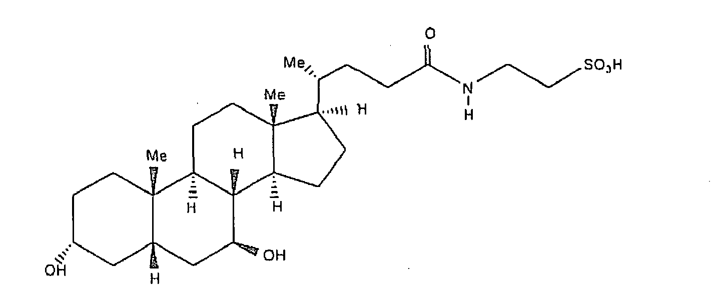 comprising the following stages:
a) obtaining an aqueous suspension of an alkali or alkaline-earth salt of tauroursodeoxycholic acid;
b) adding 0.8-1.4 equivalents of an acid, wherein said acid has a concentration in water of greater than 30% by weight;
c) leaving the resultant suspension to stand for a period of between 10 and 180 minutes;
d) filtering the suspension;
e) adding an organic solvent with subsequent precipitation of the tauroursodeoxycholic acid.

2. A process according to claim 1, **characterised in that** said aqueous suspension of an alkali or alkaline-earth salt of tauroursodeoxycholic acid is obtained by reaction between a mixed anhydride of ursodeoxycholic acid with an alkyl chloroformate and an alkali or alkaline-earth salt of taurine.

3. A process according to claim 2, **characterised in that** said aqueous suspension is obtained from the reaction of an aqueous solution of alkali metal taurinate with an acetonic solution of a mixed anhydride of ursodeoxycholic acid with a C₁-C₄ alkyl chloroformate.

4. A process according to claim 2, **characterised in that** said alkali metal laminate is sodium taurinate and/or said C₁-C₄ alkyl chloroformate is ethyl chloroformate.

5. A process according to claim 1, **characterised in that** 0.9-1.2 equivalents of acid are added in stage b), preferably 1-1.1 equivalents of acid.

6. A process according to claim 1, **characterised in that** said acid is selected from among hydrochloric acid, nitric acid, acetic acid, sulfuric acid or mixtures thereof, and/or **characterised in that** said acid has a concentration in water of between 32% and 40% by weight.

7. A process according to claim 1, **characterised in that** said acid is hydrochloric acid having a concentration in water of between 32% and 36% by weight.

8. A process according to claim 1, **characterised in that** the suspension of stage c) is left to stand for a period of between 15 and 120 minutes, preferably for a period of between 20 and 60 minutes, and/or **characterised in that** the temperature of stage c) is between 15 and 30° C.

9. A process according to claim 1, **characterised in that** said organic solvent has a water content of less than or equal to 1% by weight.

10. A process according to claim 1, **characterised in that** said organic solvent is a polar organic solvent.

11. A process according to claim 10, **characterised in that** said polar organic solvent is selected from among acetone, tetrahydrofuran, C₂-C₈ ethers, C₂-C₈ acetates and mixtures thereof, preferably is acetone.

12. A process according to claim 1, **characterised in that** said organic solvent is a nonpolar organic solvent.

13. A process according to claim 12, **characterised in that** said nonpolar organic solvent is selected from among chloroform, methylene chloride, toluene and mixtures thereof.

14. A process according to claim 1, **characterised in that** it comprises an additional purification stage by means of dissolution of the resultant tauroursodeoxycholic acid in deionised water and subsequent recrystallisation.

15. A process according to claim 14, **characterised in that** dissolution in deionised water is carried out at a temperature of less than 65°C and/or **characterised in that** dissolution in deionised water is carried out for a maximum period of 60 minutes.

16. A process according to claim 14, **characterised in that** the ratio by weight of tauroursodeoxycholic acid:deionised water is within the range from 0.1 to 1 relative to the dry weight of tauroursodeoxycholic acid.

17. A process according to claim 16, **characterised in that** the ratio by weight of tauroursodeoxycholic acid:deionised water is within the range from 0.4 to 0.6 relative to the dry weight of tauroursodeoxycholic acid.

## Patentansprüche

1. Verfahren zur Herstellung von Tauroursodesoxycholsäure mit der folgenden Formel 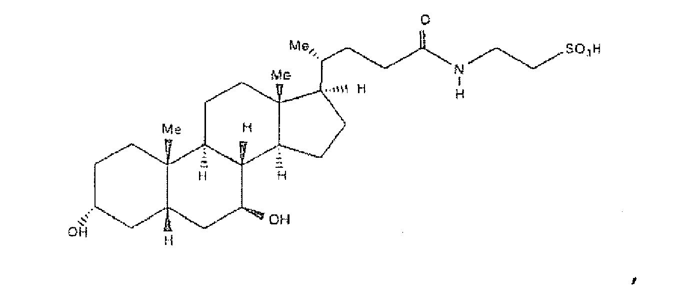 umfassend die folgenden Stufen:
a) Erhalten einer wässrigen Suspension eines Alkali- oder Erdalkali-Salzes von Tauroursodesoxycholsäure;
b) Hinzufügen von 0,8 - 1,4 Äquivalente einer Säure, wobei diese Säure eine Konzentration in Wasser von mehr als 30 Gewichtsprozent aufweist;
c) Stehenlassen der resultierenden Suspension für eine Dauer zwischen 10 und 180 Minuten;
d) Filtern der Suspension;
e) Hinzufügen eines organischen Lösungsmittels mit anschließender Präzipitation der Tauroursodesoxycholsäure.

2. Verfahren gemäß Anspruch 1, **dadurch gekennzeichnet, dass** diese wässrige Suspension eines Alkali- oder Erdalkali-Salzes der Tauroursodesoxycholsäure erhalten wird durch Reaktion zwischen einem gemischten Ursodesoxycholsäure-Anhydrid mit einem Alkyl-Chlorformiat und einem Alkali- oder Erdalkali-Salz von Taurin.

3. Verfahren gemäß Anspruch 2, **dadurch gekennzeichnet, dass** diese wässrige Suspension erhalten wird aus der Reaktion einer wässrigen Lösung eines Alkalimetall-Taurinats mit einer acetonischen Lösung eines gemischten Ursodesoxycholsäure-Anhydrids mit einem C₁-C₄ Alkyl-chlorformiat.

4. Verfahren gemäß Anspruch 2, **dadurch gekennzeichnet, dass** dieses Alkalimetall-Taurinat Natriumtaurinat und/oder dieses C₁-C₄ Alkyl-chlorformiat Ethylchlorformiat ist.

5. Verfahren gemäß Anspruch 1, **dadurch gekennzeichnet, dass** 0,9 - 1,2 Säureäquivalente bei Stufe b) hinzugefügt werden, bevorzugt 1 - 1,1 Säureäquivalente.

6. Verfahren gemäß Anspruch 1, **dadurch gekennzeichnet, dass** diese Säure ausgewählt ist aus Salzsäure, Salpetersäure, Essigsäure, Schwefelsäure oder Mischungen davon, und/oder **dadurch gekennzeichnet, dass** diese Säure eine Konzentration in Wasser zwischen 32 Gewichtsprozent und 40 Gewichtsprozent hat.

7. Verfahren gemäß Anspruch 1, **dadurch gekennzeichnet, dass** diese Säure Salzsäure mit einer Konzentration in Wasser zwischen 32 Gewichtsprozent und 36 Gewichtsprozent ist.

8. Verfahren gemäß Anspruch 1, **dadurch gekennzeichnet, dass** die Suspension von Stufe c) für eine Dauer zwischen 15 und 20 Minuten stehengelassen wird, bevorzugt für eine Dauer zwischen 20 und 60 Minuten, und/oder **dadurch gekennzeichnet, dass** die Temperatur der Stufe c) zwischen 15 und 30°C ist.

9. Verfahren gemäß Anspruch 1, **dadurch gekennzeichnet, dass** dieses organische Lösungsmittel einen Wassergehalt von weniger als oder gleich 1 Gewichtsprozent hat.

10. Verfahren gemäß Anspruch 1, **dadurch gekennzeichnet, dass** dieses organische Lösungsmittel ein polares organisches Lösungsmittel ist.

11. Verfahren gemäß Anspruch 10, **dadurch gekennzeichnet, dass** dieses polare organische Lösungsmittel ausgewählt ist aus Aceton, Tetrahydrofuran, C₂-C₈ Ether, C₂-C₈ Acetate, und Mischungen davon, bevorzugt ist Aceton.

12. Verfahren gemäß Anspruch 1, **dadurch gekennzeichnet, dass** dieses organische Lösungsmittel ein nicht-polares organisches Lösungsmittel ist.

13. Verfahren gemäß Anspruch 12, **dadurch gekennzeichnet, dass** dieses nicht-polare organische Lösungsmittel ausgewählt ist aus Chloroform, Methylenchlorid, Toluol, und Mischungen davon.

14. Verfahren gemäß Anspruch 1, **dadurch gekennzeichnet, dass** es eine zusätzliche Aufreinigungsstufe mittels Auflösen der resultierenden Tauroursodesoxycholsäure in entionisiertem Wasser und nachfolgende Rekristallisation umfasst.

15. Verfahren gemäß Anspruch 14, **dadurch gekennzeichnet, dass** die Auflösung in entionisiertem Wasser ausgeführt wird bei einer Temperatur von weniger als 65°C, und/oder **dadurch gekennzeichnet, dass** Auflösung in entionisiertem Wasser ausgeführt wird für eine maximale Dauer von 60 Minuten.

16. Verfahren gemäß Anspruch 14, **dadurch gekennzeichnet, dass** das Gewichtsverhältnis der Tauroursodesoxycholsäure :
entionisiertem Wasser innerhalb eines Bereiches von 0,1 zu 1, relativ zu dem Trockengewicht der Tauroursodesoxycholsäure, liegt.

17. Verfahren gemäß Anspruch 16, **dadurch gekennzeichnet, dass** das Gewichtsverhältnis von Tauroursodesoxycholsäure :
entionisiertem Wasser innerhalb des Bereichs von 0,4 - 0,6, relativ zu dem Trockengewicht der Tauroursodesoxycholsäure, liegt.

## Revendications

1. Procédé de préparation d'acide tauroursodésoxycholique ayant la formule suivante 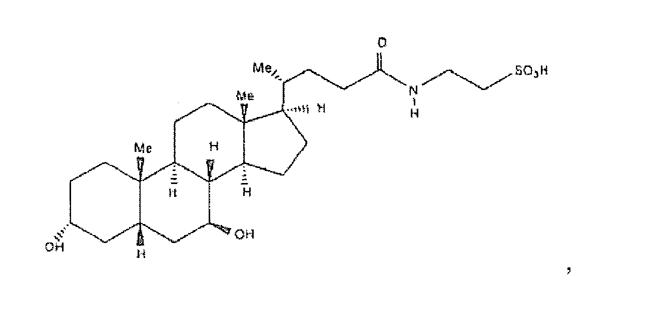 comprenant les étapes suivantes :
a) l'obtention d'une suspension aqueuse d'un sel alcalin ou alcalino-terreux d'acide tauroursodésoxycholique ;
b) l'ajout de 0,8 à 1,4 équivalent d'un acide, ledit acide ayant une concentration en eau de plus de 30 % en poids ;
c) la mise au repos de la suspension résultante pendant une période comprise entre 10 et 180 minutes ;
d) la filtration de la suspension ;
e) l'ajout d'un solvant organique avec précipitation ultérieure de l'acide tauroursodésoxycholique.

2. Procédé selon la revendication 1, **caractérisé en ce que** ladite suspension aqueuse d'un sel alcalin ou alcalino-terreux d'acide tauroursodésoxycholique est obtenue par réaction entre un anhydride d'acide tauroursodésoxycholique mélangé avec un chloroformate d'alkyle et un sel alcalin ou alcalino-terreux de taurine.

3. Procédé selon la revendication 2, **caractérisé en ce que** ladite suspension aqueuse est obtenue à partir de la réaction d'une solution aqueuse d'un taurinate de métal alcalin avec une solution acétonique d'un anhydride d'acide tauroursodésoxycholique mélangé avec un chloroformate d'alkyle en C₁-C₄.

4. Procédé selon la revendication 2, **caractérisé en ce que** ledit taurinate de métal alcalin est le taurinate de sodium et/ou ledit chloroformate d'alkyle en C₁-C₄ est le chloroformate d'éthyle.

5. Procédé selon la revendication 1, **caractérisé en ce que** 0,9 à 1,2 équivalent d'acide est ajouté à l'étape b), de préférence 1 à 1,1 équivalent d'acide.

6. Procédé selon la revendication 1, **caractérisé en ce que** ledit acide est choisi parmi l'acide chlorhydrique, l'acide nitrique, l'acide acétique, l'acide sulfurique ou leurs mélanges, et/ou **caractérisé en ce que** ledit acide a une concentration en eau comprise entre 32 % et 40 % en poids.

7. Procédé selon la revendication 1, **caractérisé en ce que** ledit acide est l'acide chlorhydrique ayant une concentration en eau comprise entre 32 % et 36 % en poids.

8. Procédé selon la revendication 1, **caractérisé en ce que** la suspension de l'étape c) est laissée au repos pendant une période comprise entre 15 et 120 minutes, de préférence pendant une période comprise entre 20 et 60 minutes, et/ou **caractérisé en ce que** la température de l'étape c) est comprise entre 15 et 30 °C.

9. Procédé selon la revendication 1, **caractérisé en ce que** ledit solvant organique a une teneur en eau inférieure ou égale à 1 % en poids.

10. Procédé selon la revendication 1, **caractérisé en ce que** ledit solvant organique est un solvant organique polaire.

11. Procédé selon la revendication 10, **caractérisé en ce que** ledit solvant organique polaire est choisi parmi l'acétone, le tétrahydrofurane, les éthers en C₂-C₈, les acétates en C₂-C₈ et leurs mélanges, de préférence l'acétone.

12. Procédé selon la revendication 1, **caractérisé en ce que** ledit solvant organique est un solvant organique non polaire.

13. Procédé selon la revendication 12, **caractérisé en ce que** ledit solvant organique non polaire est choisi parmi le chloroforme, le chlorure de méthylène, le toluène et leurs mélanges.

14. Procédé selon la revendication 1, **caractérisé en ce qu'**il comprend une étape de purification supplémentaire par dissolution de l'acide tauroursodésoxycholique résultant dans de l'eau déionisée et recristallisation ultérieure.

15. Procédé selon la revendication 14, **caractérisé en ce que** la dissolution dans de l'eau déionisée est réalisée à une température inférieure à 65 °C et/ou **caractérisé en ce que** la dissolution dans de l'eau déionisée est réalisée pendant une durée maximale de 60 minutes.

16. Procédé selon la revendication 14, **caractérisé en ce que** le rapport en poids acide tauroursodésoxycholique:eau déionisée est dans la plage allant de 0,1 à 1 par rapport au poids sec de l'acide tauroursodésoxycholique.

17. Procédé selon la revendication 16, **caractérisé en ce que** le rapport en poids acide tauroursodésoxycholique:eau déionisée est dans la plage allant de 0,4 à 0,6 par rapport au poids sec de l'acide tauroursodésoxycholique.
